# EUROPÄISCHE PATENTSCHRIFT

(11) **EP 2 461 920 B1**
(45) Veröffentlichungstag und Bekanntmachung des Hinweises auf die Patenterteilung: **29.04.2020**
(21) Anmeldenummer: 10744870.6
(22) Anmeldetag: 29.07.2010
(51) Int. Cl.: B09B 3/00, A61K 45/06, A61K 9/70

(54) **ZERSTÖRENDE ENTSORGUNG VON MEDIZINISCHEN WIRKSTOFFEN IN TRANSDERMALEN THERAPEUTISCHEN SYSTEMEN**
DESTRUCTIVE DISPOSAL OF MEDICAL ACTIVE INGREDIENTS IN TRANSDERMAL THERAPEUTIC SYSTEMS
ELIMINATION DESTRUCTIVE DE PRINCIPES ACTIFS MÉDICAUX DANS DES SYSTÈMES THÉRAPEUTIQUES TRANSDERMIQUES

(30) Priorität: 07.08.2009 DE 102009036485
(43) Veröffentlichungstag der Anmeldung: 13.06.2012
(73) Patentinhaber: LTS LOHMANN Therapie-Systeme AG, 56626 Andernach (DE)
(72) Erfinder: WIRZ, Margit, 56751 Polch (DE); HILLE, Thomas, 56567Neuwied (DE)
(74) Vertreter: Schweitzer, Klaus
(86) Internationale Anmeldenummer: PCT/EP2010/004641
(87) Internationale Veröffentlichungsnummer: WO 2011/015308

(56) Entgegenhaltungen:
- WO-A1-03/103673
- WO-A2-2005/041883
- WO-A2-2007/137732
- US-A- 4 510 621
- US-A1- 2005 163 717

## Beschreibung

Die Erfindung betrifft ein Mittel und ein Verfahren zur zerstörenden Entsorgung von medizinischen Wirkstoffen, nachfolgend auch kurz als Arzneiform bezeichnet, die in transdermalen therapeutischen Systemen (= TTS), ggf. auch als transdermale Pflaster bezeichnet, enthalten sind. Das erfindungsgemäße Mittel wird mit dem TTS nach. Gebrauch in Kontakt gebracht, wodurch die Arzneiform in einer chemischen Reaktion zerstört oder zersetzt, auf jeden Fall aber ihrer medizinischen Wirkung beraubt wird. Das TTS selbst enthält normalerweise als Arzneiform einen therapeutischen Wirkstoff, bevorzugt einen aus der Gruppe der Schmerzmittel, der aus dem System durch Diffusion an die Haut herangetragen und dann transdermal zu therapeutischen Zwecken appliziert wird.

Transdermale Applikationen mit den medizinischen Wirkstoffen Buprenorphin und Fentanyl sind die Arzneiformen der Wahl zur Behandlung von chronischen Schmerzen in der Langzeittherapie. Durch die kontinuierliche Abgabe von solchen hochwirksamen Schmerzmitteln über die Haut wird ein Schmerzpatient kontinuierlich über längere Zeit hinweg mit einer konstanten Dosis an Schmerzmittel versorgt, so dass Plasmaspitzen und Plasmatäler vermieden werden. Das hat den Vorteil, dass durch eine niedrige aber ausreichende Plasmakonzentration des Wirkstoffs sowohl Nebenwirkungen durch Überdosierungen, aber auch vermeidbare Schmerzzustände durch Unterversorgung, nicht auftreten. Dem Fachmann sind beispielsweise die Handelsprodukte Transtec®, aber auch Durogesic® oder Durogesic® Smat bekannt, die sich schon seit längerer Zeit in der Schmerztherapie bestens bewährt haben.

Der Nachteil der TTS in der Schmerztherapie besteht allerdings darin, dass zur Aufrechterhaltung des so genannten Konzentrationsgradienten und damit des für die Therapie gewünschten Plasmaspiegels des medizinischen Wirkstoffs über die gesamte Zeitdauer der Applikation der TTS stets eine größere Vorratsmenge an Wirkstoff im TTS enthalten sein muss als tatsächlich an den Patienten abgegeben wird. Das hat zur Folge, dass getragene oder gebrauchte TTS ein Missbrauchspotential für z.B. Angehörige der Drogenszene darstellen. Diese Personengruppen sind durchaus in der Lage, getragene TTS zu sammeln und den noch vorhandenen, restlichen medizinischen Wirkstoff zu extrahieren und ihn zur Befriedigung der Drogensucht missbräuchlich einzunehmen.

Sinngemäß gelten solche Überlegungen auch für das Hormon Testosteron oder das Sympathomimeticum Methylphenidat. Beide Arzneistoffe werden zur Vermeidung von Plasmaspitzen und -tälern vorteilhaft transdermal appliziert und beinhalten ein gewisses Missbrauchspotential, weshalb z.B. Methylphenidat weltweit als Narkoticum angesehen wird. Testosteron unterliegt in den USA ähnlichen Rechtsvorschriften wie Narkotica.

In der Vergangenheit hat es daher nicht an Versuchen gemangelt, diesen unkontrollierten Missbrauch dadurch zu unterbinden, dass Patienten geraten worden ist, getragene Pflaster zu zerschneiden und sie dann durch die Toilette der Kanalisation zuzuführen. Nachteilig an diesem Verfahren ist, dass die massenweise Entsorgung von Medikamenten durch die Kanalisation ein nicht zu unterschätzendes Umweltproblem darstellt. Darüber hinaus besteht die Gefahr, dass Abflussrohre durch schwer lösliche Trägermaterialien der TTS verstopft werden.

In der Folge wurden TTS entwickelt, die neben dem Wirkstoff zugleich auch einen Antagonisten enthielten (z.B. WO 2004/098576, WO 90/04965, WO 2004/037259). Dadurch sollte die oben beschriebene Gewinnung bzw. missbräuchliche Extraktion des medizinischen Wirkstoffs aus gebrauchten TTS verhindert, zumindest aber doch deutlich erschwert werden. Diese Schutzmaßnahmen haben sich jedoch als nicht ausreichend zur Vermeidung von Medikamentenmissbrauch erwiesen, weil nach wie vor theoretisch und sogar mit relativ einfachen Mitteln der eigentliche medizinische Wirkstoff durch fraktionierte Fällung von dem Antagonisten getrennt werden kann.

Die WO 2007/137732 beschreibt ein TTS, das neben einem Wirkstoff noch ein davon abgetrenntes, den Wirkstoff unbrauchbar machendes Agens in einer Lösung enthält. Zusätzlich dazu ist noch ein Mittel vorhanden, das nach Gebrauch des TTS den Weg frei macht, damit das Agens mit dem Wirkstoff in Kontakt tritt und diesen unbrauchbar macht. Der Nachteil an dieser ansonsten perfekten Lösung besteht aber darin, dass das Agens in Lösung wegen seiner hohen Reaktivität die Lagerfähigkeit einschränkt und dass teilweise auch beim Transport die Gefahr von Beschädigung durch Flüssigkeitsaustritt vorhanden ist.

Die nicht vorveröffentlichte DE 10 2008 016 804 schlägt bereits ein TTS vor, das sich nach Gebrauch, d.h. nach seiner Ablösung von der Hautoberfläche des Patienten durch den Patienten, selbst zerstört. Sich selbst zerstörendes TTS im Sinne dieser früheren Entwicklung bedeutet, dass der in dem TTS enthaltene restliche medizinische Wirkstoff nach Gebrauch direkt oder indirekt zerstört, chemisch zersetzt und/oder unbrauchbar gemacht wird. Bei der vorgeschlagenen Ausführungsform wurde auch möglichst gewährleistet, dass der Zerstörungsprozess nicht schon vor oder schon während der transdermalen Applikation des TTS einsetzt. Nachteilig an dieser Ausführungsform ist jedoch die technisch aufwändige Fertigungsweise, die aus ökonomischer Sicht ein Problem darstellt.

Das Dokument WO-A1-03/103673 offenbart ein Mittel und ein Verfahren zur zerstörenden Entsorgung von medizinischen Wirkstoffen, die in transdermalen therapeutischen Systemen enthalten sind, gemäß dem Oberbegriff des Anspruchs 1, bzw. des Anspruchs 7.

Der vorliegenden Erfindung lag daher die Aufgabe zu Grunde, ein Mittel bereitzustellen, mit dem nach bestimmungsgemäßem Gebrauch eines TTS eine missbräuchliche Entnahme von restlichem medizinischen Wirkstoff zuverlässig und vollständig unterbunden wird. Zusätzlich soll das Mittel einfach herzustellen und über eine längere Zeitdauer problemlos lagerfähig sein. Darüber hinaus soll das Mittel vom Anwender des TTS bei hoher Zuverlässigkeit seiner Wirkung auch noch leicht zu handhaben sein.

Gelöst wird diese Aufgabe durch das Mittel gemäß Anspruch 1 und das Verfahren gemäß Anspruch 7.

Das erfindungsgemäße Mittel enthält in einer bevorzugten Ausführungsform zusätzlich noch eine Schutzschicht. Es wird getrennt von der Arzneiform aufbewahrt. Das getrennt von der Arzneiform aufbewahrte erfindungsgemäße Mittel wird nach dem Gebrauch der Arzneiform mit dieser in Kontakt gebracht.

Bei dem Agens, das in die Schicht des offenbarungsgemäßen Mittels eingelagert ist, kann es sich um eine Substanz oder um ein Substanzgemisch handeln, die oder das erfindungsgemäß als Feststoff oder als Paste vorliegen kann. Bevorzugt ist das Agens eine Substanz, die mit dem medizinischen Wirkstoff, der Arzneiform, chemisch reagiert und ihn dadurch zerstört. Diese Wirkung erreichen insbesondere chemische Oxidationsmittel wie z.B. anorganische Reagenzien, wie Permanganate, z.B. Kaliumpermanganat, Mangandioxid, Bleidioxid, Bleitetraacetat, Cer(IV)-Salze, Chromate, Osmiumtetroxid, Nitrite, wie Kaliumnitrit, Selendioxid, PeroxoVerbindungen, Hypohalogenide, oder Schwefel; bevorzugt von diesen sind Kaliumpermanganat und Kaliumnitrit. Organische Oxidantien, wie Dimethylsulfoxid, N-Bromsuccinimid, Chinone, hypervalente Iodverbindungen, Persäuren und Perester, aber auch Enzyme können ebenso zum Einsatz kommen. Bei gegebenem medizinischen Wirkstoff wird das Agens bevorzugt auf Grund seiner chemischen Reaktionsfähigkeit mit dem Wirkstoff ausgewählt. Dem Fachmann ist im allgemeinen bekannt, welches Agens für welche Arzneiform im erfindungsgemäßen Sinne am besten geeignet ist. Erfindungsgemäß ist das Agens Kaliumpermanganat oder Kaliumnitrit, welches in fester oder pastöser Form vorliegt.

Bei dem medizinischen Wirkstoff handelt es sich bevorzugt um einen Wirkstoff aus der Gruppe der Schmerzmittel wie z. B. Narkotika. Bevorzugt sind zu nennen Morphinderivate, Heroin und Buprenorphin, oder Fentanyl und seine Derivate Sufentanil und Alfentanil. Grundsätzlich sind auch alle anderen Kombinationen von Wirkstoff und Agens verwendbar, für die eine transdermale Applikation über ein TTS eine geeignete Darreichungsform ist, z.B. Testosteron und Methlyphenidat.

Das erfindungsgemäße Mittel mit mehrschichtigem Aufbau enthält mindestens eine Schicht, in die das Agens eingelagert ist. Erfindungsgemäß handelt es sich dabei um eine polymere Schicht oder um eine Haftkleberschicht. Als Polymere können hierzu Standardpolymere eingesetzt werden wie z.B. Polyamid, Polyimid, Polytetrafluorethylen, Polyethylen, Polypropylen, Polyvinylchlorid, Polyacrylate oder Polymethacrylate, Polystyrol oder Polyester. Die Einlagerung erfolgt so, dass das Agens sich aus der polymeren Schicht leicht wieder herauslösen lässt, um seinem bestimmungsgemäßen Zweck, die zerstörende Entsorgung der Arzneiform, nachzukommen.

Als Haftkleberschicht kommen Klebstoffe zum Einsatz, die ohne Lösungsmittel bei Raumtemperatur permanent klebrig sind oder klebfähig bleiben und bei leichtem Anpressdruck auf praktisch allen Substraten haften. Basis für geeignete Haftkleber sind vorzugsweise natürliche oder synthetische Kautschuke, Polyacrylate, Polyester, Polychloroprene, Polyisobutene, Polyvinylether oder Polyurethane, die normalerweise in Kombination mit natürlichen oder synthetischen Harzen und mit Oxidationsstabilisatoren für ihren bestimmungsgemäßen Zweck eingesetzt werden. Im Fall der Haftkleberschicht erfolgt die Einlagerung des Agens durch Aufstreuen. So lässt sich das Agens aus der Haftkleberschicht leicht wieder herauslösen.

Das erfindungsgemäße Mittel mit mehrschichtigem Aufbau enthält weiter mindestens ein Gewebe, ein Gewirke oder ein Vlies aus mineralischen Fasern, wie z.B. Glas, Mineralwolle, Basalt, tierischen Fasern wie z.B. Seide oder Wolle, pflanzlichen Fasern wie z.B. Baumwolle oder chemischen Fasern aus natürlichen (z.B. Cellulose) und/oder synthetischen Polymeren. Als synthetische Kunststoffe können hierzu ebenso Standardpolymere eingesetzt werden wie für die polymere Schicht, nämlich Polyamid, Polyimid, Polytetrafluorethylen, Polyethylen, Polypropylen, Polyvinylchlorid, Polyacrylate oder Polymethacrylate, Polystyrol oder Polyester.

Das erfindungsgemäße Mittel enthält in einer bevorzugten Ausführungsform zusätzlich noch eine Schutzschicht. Die Schutzschicht ist dabei auf der freien Oberfläche der Polymerschicht angeordnet, das ist die der Faserschicht gegenüberliegende Seite. Als Schutzschicht kommen erfindungsgemäß insbesondere Folien aus Kunststoff zum Einsatz, z.B. aus Polyethylen, aus Polypropylen oder aus Polyester.

Das erfindungsgemäße Mittel kann zusätzlich eine weitere Kleberschicht zur Fixierung der Schutzschicht enthalten, was insbesondere dann zweckmäßig ist, wenn die Schicht, in die das Agens eingelagert ist, nicht selbst eine Haftkleberschicht ist.

Das getrennt von der Arzneiform aufbewahrte, erfindungsgemäße Mittel mit mehrschichtigem Aufbau wird zu seinem bestimmungsgemäßen Einsatzzweck, der zerstörenden Entsorgung des medizinischen Wirkstoffs, mit der Arzneiform in Kontakt gebracht. Das geschieht so, dass nach dem Abnehmen des transdermalen Pflasters/TTS von der Haut des Patienten die Faserschicht des erfindungsgemäßen Mittels mit einer geringen Menge Flüssigkeit angefeuchtet wird. Dann wird das getragene TTS auf die angefeuchtete Faserschicht des erfindungsgemäßen Mittels mit mehrschichtigem Aufbau aufgeklebt. Die Flüssigkeit tritt durch die Faserschicht hindurch an das Agens heran, das in der polymeren Schicht eingelagert ist, löst es dort heraus und bewirkt so, dass das Agens durch die Faserschicht zurück diffundiert und so mit dem medizinischen Wirkstoff in unmittelbaren Kontakt kommt und ihn dabei chemisch zerstört.

Das erfindungsgemäße Mittel zur zerstörenden Entsorgung von medizinischen Wirkstoffen in TTS mit mehrschichtigem Aufbau eignet sich für alle bekannten TTS, für deren Herstellung der Fachmann grundsätzlich auf die Materialien, Herstellungsverfahren und den Aufbau der aus dem Stand der Technik bekannten TTS bzw. transdermalen Pflaster zurückgreift (vgl. dazu: Transdermale Pflaster; Spektrum der Wissenschaft 10/2003, 42; Transdermal Controlled Systemic Madications, Y.W. Chien, Drugs and the Pharmaceutical Sciences, Vol. 31; Polymers in Transdermal Drug Delivery Systems, S. Kandavilli et. al., Pharmaceutical Technology, May 2002, 62-80).

Die Erfindung wird durch das nachfolgende Ausführungsbeispiel näher erläutert ohne auf die darin beschriebene, individuelle Ausführungsform und Stoffauswahl beschränkt zu sein. Gleichwohl können spezielle, in den Beispielen beschriebene Ausgestaltungen des erfindungsgemäßen Mittels zur zerstörenden Entsorgung von medizinischen Wirkstoffen in TTS einzeln oder in Kombination miteinander als bevorzugte Merkmale für die Erfindung als solche verallgemeinert werden.

### Beispiel 1

In Ethylacetat wurde ein solcher Anteil von neutralem Polymethacrylat (Plastoid B) gelöst, dass eine Polymerlösung mit 40 Gew.-% Feststoffanteil entstand. Nach vollständiger Auflösung des Polymers wurde der gleiche Gewichtsanteil an feinkörnigem Kaliumpermanganat bei Raumtemperatur unter Rühren hinzususpendiert. Die so erhaltene Polymerlösung wurde mit einem geeigneten Streichrakel auf eine silikonisierte Polyesterfolie aufgetragen. Danach wurde das Lösemittel in einem dem Fachmann bekannten Labortrockenschrank bei einer Temperatur von 60 °C entfernt. Nach dem Entfernen des Lösemittels betrug das Flächengewicht 180 g/m².

In einem maschinellen Prozess wurden aus der getrockneten Laminatbahn Einzelsegmente ausgestanzt, die annähernd die Größe von handelsüblichen Transtec® TTS aufwiesen (70 mal 70 mm). Abschließend wurde die polymere Schicht mit eingelagertem Kaliumpermanganat mit einem Wirrfaservlies aus Cellulosefasern abgedeckt.

Als Test wurden jeweils drei so hergestellte erfindungsgemäße Mittel mit Mehrschichtstruktur an TTS enthaltend als medizinischen Wirkstoff Buprenorphin über verschiedene Einwirkzeiten hinweg ausprobiert. Dazu wurde das Vlies mit 4,5 ml Wasser angefeuchtet und auf das angefeuchtete Vlies wurde das TTS aufgeklebt. Nach der in der nachfolgenden Tabelle angegebenen Einwirkzeit wurde die Trennung des TTS von dem Vlies vorgenommen und das restliche Buprenorphin mit Isopropanol plus 0,1 % Ascorbinsäure herausgelöst. Die Bestimmung der Menge an restlichem Wirkstoff wurde durch Hochdruckflüssigkeitschromatographie (HPLC) vorgenommen.

Die Ergebnisse sind in der nachfolgenden Tabelle zusammengestellt.

| **Muster** | **Einwirkzeit** | **Gehalt [mg/TTS]** | **Wiederfindung [%]** |
|---|---|---|---|
| Bph20 als Vergleich | ./. | 20,3 | 101,3 |
| Bph20 | 0,5 h | 17,3 | 86,3 |
| | 1 h | 16,1 | 80,7 |
| | 2 h | 10,5 | 52,5 |
| | 3 h | 2,7 | 13,3 |
| | 5 h | 0,2 | 1,0 |
| | 6 h | 0,2 | 1,0 |

Es zeigte sich deutlich, dass nach einer Einwirkzeit von 3 bis 4 Stunden keine nennenswerte Menge an medizinischem Wirkstoff mehr nachzuweisen war. Somit hat das erfindungsgemäße Mittel seinen Zweck der zerstörenden Entsorgung vollständig erreicht und damit eine missbräuchliche und zweckentfremdete Wiederverwendung wirksam unterbunden.

## Patentansprüche

1. Mittel zur zerstörenden Entsorgung von medizinischen Wirkstoffen, die in transdermalen therapeutischen Systemen TTS enthalten sind, das getrennt von dem zur zerstörenden Entsorgung vorgesehenen medizinischen Wirkstoff aufbewahrbar und nach dem Gebrauch des TTS mit diesem in Kontakt bringbar ist, wobei das Mittel einen mehrschichtigen Aufbau besitzt, und wenigstens eine Schicht mit darin eingelagertem Agens enthält, wobei die Schicht mit darin eingelagertem Agens eine polymere Schicht mit darin eingelagertem Agens oder eine Haftkleberschicht mit darin eingelagertem Agens ist,
**dadurch gekennzeichnet, dass** das Mittel
wenigstens ein Gewebe, ein Gewirke oder ein Vlies enthält aus mineralischen Fasern, insbesondere Glas, Mineralwolle, Basalt, aus tierischen Fasern wie Seide oder Wolle, aus pflanzlichen Fasern, insbesondere Baumwolle oder aus chemischen Fasern aus natürlichen oder synthetischen Polymeren, insbesondere Polyamid, Polyimid, Polytetrafluorethylen, Polyethylen, Polypropylen, Polyvinylchlorid, Polyacrylat oder Polymethacrylat, Polystyrol oder Polyester, wobei das Agens Kaliumpermanganat oder Kaliumnitrit ist, welches in fester oder in pastöser Form vorliegt.

2. Mittel nach Anspruch 1, **dadurch gekennzeichnet, dass** es zusätzlich noch eine Schutzschicht enthält.

3. Mittel nach einem der Ansprüche 1 oder 2, **dadurch gekennzeichnet, dass** die Schicht mit darin eingelagertem Agens eine polymere Schicht ist und Standardpolymere enthält, insbesondere Polyamid, Polyimid, Polytetrafluorethylen, Polyethylen, Polypropylen, Polyvinylchlorid, Polyacrylate oder Polymethacrylate, Polystyrol oder Polyester.

4. Mittel nach einem der Ansprüche 1 bis 3, **dadurch gekennzeichnet, dass** die Schicht mit darin eingelagertem Agens eine Haftkleberschicht ist und Haftkleber auf Basis von natürlichem oder synthetischem Kautschuk, von Polyacrylat, von Polyester, von Polychloropren, von Polyisobuten, von Polyvinylether oder von Polyurethan enthält, die vorzugsweise in Kombination mit natürlichen oder synthetischen Harzen und mit Oxidationsstabilisatoren eingesetzt werden.

5. Mittel nach einem der Ansprüche 1 bis 4, **dadurch gekennzeichnet, dass** es eine Schutzschicht enthält, die auf der freien Oberfläche der Schicht mit darin eingelagertem Agens, die der Faserschicht gegenüberliegt, angeordnet ist.

6. Mittel nach einem der Ansprüche 1 bis 5, **dadurch gekennzeichnet, dass** es als Schutzschicht Folien aus Kunststoff, insbesondere Polyethylen, Polypropylen oder Polyester enthält.

7. Verfahren zur zerstörenden Entsorgung von medizinischen Wirkstoffen, die in transdermalen therapeutischen Systemen enthalten sind, **dadurch gekennzeichnet, dass** ein transdermal therapeutisches System von der Haut des Patienten abgenommen wird, die Faserschicht des Mittels zur zerstörenden Entsorgung von medizinischen Wirkstoffen nach einem der Ansprüche 1 bis 6 mit Flüssigkeit angefeuchtet wird und das abgenommene transdermale therapeutische System auf die Faserschicht des Mittels zur zerstörenden Entsorgung von medizinischen Wirkstoffen aufgeklebt wird.

## Claims

1. Means for the destructive disposal of medical active ingredients present in transdermal therapeutic systems TTS, which means can be stored separate from the medical active ingredient intended for destructive disposal and can be contacted with the TTS after said TTS has been used, wherein the means possesses a multilayer construction, and comprises at least one layer with agent incorporated therein, wherein the layer with agent incorporated therein is a polymeric layer with agent incorporated therein or a pressure-sensitive adhesive layer with agent incorporated therein, **characterized in that** the means comprises at least one woven fabric, a knitted fabric or a nonwoven fabric made from mineral fibers, in particular glass, mineral wool, basalt, from animal fibers such as silk or wool, from plant fibers, in particular such as cotton or from chemical fibers composed of natural or synthetic polymers, in particular polyamide, polyimide, polytetrafluoroethylene, polyethylene, polypropylene, polyvinyl chloride, polyacrylate or polymethacrylate, polystyrene or polyester, wherein the agent is potassium permanganate or potassium nitrite in solid or pastelike form.

2. Means according to Claim 1, **characterized in that** it further comprises a protective layer.

3. Means according to either of Claims 1 and 2, **characterized in that** the layer with agent incorporated therein is a polymeric layer and comprises standard polymers, in particular polyamide, polyimide, polytetrafluoroethylene, polyethylene, polypropylene, polyvinyl chloride, polyacrylates or polymethacrylates, polystyrene or polyesters.

4. Means according to any of Claims 1 to 3, **characterized in that** the layer with agent incorporated therein is a pressure-sensitive adhesive layer and comprises pressure-sensitive adhesives based on natural or synthetic rubber, on polyacrylate, on polyester, on polychloroprene, on polyisobutene, on polyvinyl ether or on polyurethane, which are used preferably in combination with natural or synthetic resins and with oxidation stabilizers.

5. Means according to any of Claims 1 to 4, **characterized in that** it comprises a protective layer which is disposed on the free surface of the layer with agent incorporated therein, which is opposite the fiber layer.

6. Means according to any of Claims 1 to 5, **characterized in that** it comprises as protective layer films of plastic, in particular polyethylene, polypropylene or polyester.

7. Method for the destructive disposal of medical active ingredients present in transdermal therapeutic systems, **characterized in that** a transdermally therapeutic system is removed from the patient's skin, the fiber layer of the means for the destructive disposal of medical active ingredients according to any of Claims 1 to 6 is moistened with liquid, and the transdermal therapeutic system removed is adhered to the fiber layer of the means for the destructive disposal of medical active ingredients.

## Revendications

1. Moyen d'élimination destructive de principes actifs médicaux qui sont contenus dans des systèmes thérapeutiques transdermiques TTS qui peut être conservé séparément du principe actif médical prévu pour l'élimination destructive et peut être amené en contact après l'usage du TTS avec celui-ci, dans lequel le moyen possède une structure à couches multiples, et contient au moins une couche avec un agent incorporé à l'intérieur, dans lequel la couche avec un agent incorporé à l'intérieur est une couche polymère avec un agent incorporé à l'intérieur ou une couche adhésive autocollante avec un agent incorporé à l'intérieur,
**caractérisé en ce que** le moyen contient au moins un tissu, une maille ou un non-tissé de fibres minérales, en particulier du verre, de la laine minérale, du basalte, des fibres animales telles que la soie ou la laine, des fibres végétales, en particulier du coton ou des fibres chimiques de polymères naturels ou synthétiques, en particulier du polyamide, du polyimide, du polytétrafluoroéthylène, du polyéthylène, du polypropylène, du chlorure de polyvinyle, du polyacrylate ou du polyméthacrylate, du polystyrène ou du polyester, dans lequel l'agent est un permanganate de potassium ou nitrite de potassium qui se présente sous la forme solide ou pâteuse.

2. Moyen selon la revendication 1, **caractérisé en ce qu'**il contient en outre encore une couche de protection.

3. Moyen selon l'une quelconque des revendications 1 ou 2, **caractérisé en ce que** la couche avec un agent incorporé à l'intérieur est une couche polymère et contient des polymères standard, en particulier du polyamide, du polyimide, du polytétrafluoroéthylène, du polyéthylène, du polypropylène, du chlorure de polyvinyle, du polyacrylate ou du polyméthacrylate, du polystyrène ou du polyester.

4. Moyen selon l'une quelconque des revendications 1 à 3, **caractérisé en ce que** la couche avec un agent incorporé à l'intérieur est une couche adhésive autocollante et contient un adhésif autocollant à base de caoutchouc naturel ou synthétique, de polyacrylate, de polyester, de polychloroprène, de polyisobutène, de polyvinyléther ou de polyuréthane qui sont de préférence utilisés en combinaison avec des résines naturelles ou synthétiques et avec des stabilisants à l'oxydation.

5. Moyen selon l'une quelconque des revendications 1 à 4, **caractérisé en ce qu'**il contient une couche de protection qui est agencée sur la surface libre de la couche avec un agent incorporé à l'intérieur qui fait face à la couche de fibre.

6. Moyen selon l'une quelconque des revendications 1 à 5, **caractérisé en ce qu'**il contient comme couche de protection des feuilles en matière plastique, en particulier du polyéthylène, du polypropylène ou du polyester.

7. Procédé d'élimination destructive de principes actifs médicaux qui sont contenus dans des systèmes thérapeutiques transdermiques, **caractérisé en ce qu'**un système thérapeutique transdermique est retiré de la peau du patient, la couche de fibre du moyen est humidifiée avec du liquide pour l'élimination destructive de principes actifs médicaux selon l'une quelconque des revendications 1 à 6 et le système thérapeutique transdermique retiré est collé sur la couche de fibre du moyen pour l'élimination destructive de principes actifs médicaux.
